# EUROPÄISCHE PATENTSCHRIFT

(11) **EP 1 946 692 B1**
(45) Veröffentlichungstag und Bekanntmachung des Hinweises auf die Patenterteilung: **14.03.2018**
(21) Anmeldenummer: 08000347.8
(22) Anmeldetag: 10.01.2008
(51) Int. Cl.: A47L 15/50

(54) **Spülmaschine und Spülgutträger mit einer Kupplungsvorrichtung zur Einleitung von Spül- und oder Desinfektionsflüssigkeit und/oder Trocknungsluft**
Dishwasher and basket with coupling device for admission of cleaning and or desinfection agent and/or Drying air
Lave-vaisselle et panier avec système d'accouplement pour l'admission de produit de lavage et ou désinfectant et /ou d'air de séchage

(30) Priorität: 19.01.2007 DE 102007003894
(43) Veröffentlichungstag der Anmeldung: 23.07.2008
(73) Patentinhaber: Miele & Cie. KG, 33332 Gütersloh (DE)
(72) Erfinder: Dyck, Heinrich, 33719 Bielefeld (DE); Knorr, Jochen, 33659 Bielefeld (DE)

(56) Entgegenhaltungen:
- EP-A2- 0 796 587
- WO-A1-83/01892
- DE-A1- 19 705 548
- DE-B3-102004 022 025
- FR-A1- 2 508 789

## Beschreibung

Die Erfindung betrifft eine Spülmaschine, insbesondere Reinigungs- und/oder Desinfektionsautomat für schlauchförmiges Reinigungsgut, mit einem Spülraum zur Aufnahme von mindestens einem Spülgutträger, und mit einer Kupplungsvorrichtung zur Einleitung von Spül- und oder Desinfektionsflüssigkeit und/oder Trocknungsluft in ein am Spülgutträger angeordnetes Anschlussstück. Die Erfindung betrifft außerdem einen Spülgutträger zum Einschub in den Spülraum einer Spülmaschine, insbesondere eines Reinigungs- und/oder Desinfektionsautomaten für schlauchförmiges Reinigungsgut, mit einem Anschlussstück zur Verbindung mit einer Kupplungsvorrichtung der Spülmaschine zur Einleitung von Spül- und oder Desinfektionsflüssigkeit und/oder Trocknungsluft, **wobei das Anschlussstück relativ zu seinem Befestigungsbereich am Spülgutträger beweglich angeordnet ist.** Daneben betrifft die Erfindung ein System aus einer Spülmaschine und einem Spülgutträger.

Eine Spülmaschine, ein Spülgutträger und ein System der eingangs genannten Art sind aus der DE 103 00 501 A1 bekannt. Als Kupplungsvorrichtung wird bei der bekannten Spülmaschine eine Aufnahme mit einem Dichtschlauch verwendet, als Anschlussstück ein Rohrstutzen. Eine solche sehr einfach gehaltene Kupplung bedingt eine Anordnung der Aufnahme an der Rückseite des Spülraums.

Aus der DE 103 17 150 A1 ist eine automatische Kupplungsvorrichtung bekannt, bei der ein Dichtungselement mittels Druckluft in Richtung des Anschlussstücks bewegt wird. Eine solche Dichtung bedingt eine permanente Aufrechterhaltung des Überdrucks und außerdem eine sehr exakte Vorzentrierung der miteinander zu kuppelnden Teile.

Die EP 0 483 059 B1 zeigt eine Spülmaschine, in der eine Pneumatikeinrichtung die gesamte Kupplungseinrichtung in Richtung Anschlussstück (Korbwandung mit Stutzen) verschiebt. Hierdurch entstehen einerseits Probleme bei der Abdichtung der Kupplungseinrichtung gegenüber der Spülraumwand. Andererseits muss der Spülgutträger und seine gesamte Lagerung nahezu spielfrei im Spülraum aufgenommen werden, um ein Ausweichen des Anschlussstücks gegenüber der Kupplungseinrichtung zu vermeiden.

### Aus der EP 0 762 587 A2 ist eine Haushalts-Geschirrspülmaschine bekannt, bei der an einem Geschirrkorb ein Sprüharm und dessen Zulaufrohr befestigt ist. Das Zulaufrohr ist über zwei Öffnungen mit einem spülmaschinenseitigen Anschlussstutzen gekuppelt. Dabei ist es in der Höhe verstellbar.

Der Erfindung stellt sich somit das Problem, eine Spülmaschine, einen Spülgutträger bzw. ein System aus diesen beiden Komponenten zu offenbaren, bei dem einerseits Freiheit in der Wahl der Kupplungsstelle besteht und andererseits eine einfache, aber sicher dichtende Ankopplung gewährleistet ist.

Erfindungsgemäß wird dieses Problem durch eine Spülmaschine, einen Spülgutträger und insbesondere durch ein System mit den Merkmalen der unabhängigen Patentansprüche gelöst. Vorteilhafte Ausgestaltungen und Weiterbildungen der Erfindung ergeben sich aus den jeweils nachfolgenden Unteransprüchen.

Die mit der Erfindung erreichbaren Vorteile bestehen einerseits in einer sehr einfach aufgebauten Anordnung, welche aufgrund ihrer Selbstzentrierung nur geringe Anforderungen an Toleranzen in der Lagerung des Spülgutträgers im Spülraum stellt. Andererseits können selbsthemmende Antriebe verwendet werden, welche nach der Ankupplung abgeschaltet werden können.

In einer vorteilhaften Ausführungsform ist die Zugeinrichtung innerhalb der Kupplungsvorrichtung angeordnet. Hierdurch ergibt sich eine kompakte, vorprüfbare Einheit, bei der die Zugkräfte in der Nähe der abzudichtenden Bauteile angreifen und deshalb große Wirkung entfalten. Dabei umfasst die Zugeinrichtung in zweckmäßiger Weise einen elektromotorisch oder elektromagnetisch bewegbaren Kupplungsdorn, welcher mit dem Spülgutträger, insbesondere mit dem Anschlussstück kuppelbar ist. Der Kupplungsdorn kann in vorteilhafter Weise durch einen Kurbeltrieb bewegt werden. Der Kurbeltrieb bietet im Totpunkt ein Höchstmaß an Selbsthemmung und gewährleistet somit einen sicheren Erhalt der Dichtungskräfte auch bei Abschaltung seines Antriebsmotors nach Erreichen der Kupplungsposition. Der Kupplungsdorn kann eine Spülraumwand wenigstens teilweise durchragen. Dann ist es vorteilhaft, wenn der Kupplungsdorn von einer Membran umgeben ist, welche die Durchtrittsöffnung in der Spülraumwand abdichtet. Die Verwendung einer Membran anstelle von schleifenden Dichtungsteilen gewährleistet Verschleißfreiheit. Es ist besonders vorteilhaft, wenn die Membran zu einer Dichtfläche erweitert ist, die an der Spülrauminnenseite der vor den Enden von Kupplungsstutzen der Kupplungsvorrichtung liegt. Hierdurch wird erreicht, dass ein einstückiges Bauteil alle Dichtfunktionen erfüllt, nämlich
- die Abdichtung des Kupplungsdorns gegenüber der Spülraumwand;
- die Abdichtung der Zuleitungen der Kupplungsvorrichtung gegenüber der Spülraumwand;
- die Abdichtung der beiden Leitungspartner von Kupplungsvorrichtung und Anschlussstück gegeneinander.

Insbesondere bei einer sogenannten Durchschubmaschine, d. h. bei einer Spülmaschine mit zwei gegenüber angeordneten Spülraumöffnungen ist es zweckmäßig, die Kupplungsvorrichtung in einer Seitenwand des Spülraums anzuordnen.

Um das angekuppelte Anschlussstück in seinen dichtenden Sitz an der Kupplungseinrichtung heranziehen zu können, ist es relativ zu seinem Befestigungsbereich am Spülgutträger beweglich angeordnet. Hierdurch wird in einfacher Weise und mit geringem Kraftaufwand der Kontakt zwischen den beiden Dichtungspartnern hergestellt. Denkbar wäre auch eine feste Anordnung des Anschlussstücks am Spülgutträger, verbunden mit einer Vorrichtung zur Verschiebung des gesamten Spülgutträgers. Hier besteht dann allerdings insbesondere bei einer seitlichen Anordnung der Kupplungsvorrichtung die Gefahr, dass der Spülgutträger beim Heranziehen verkantet. Eine solche Ausgestaltung bietet sich deshalb eher an, wenn die Kupplungsvorrichtung an der Spülraumrückwand angeordnet ist, da dann bereits vorhandene Einrichtungen zum Einschieben des Spülgutträgers in den Spülraum genutzt werden können. Bei einer Anordnung der Kupplungsvorrichtung an einer Spülraumseitenwand ist es aus den vorgenannten Gründen einfacher und damit vorteilhafter, wenn das Anschlussstück quer zur Einschubrichtung des Spülgutträgers zur Kupplungsvorrichtung hin beweglich ist. In allen Fällen ergibt sich eine einfache Platzierung des Anschlussstücks vor der Kupplungsvorrichtung durch Verwendung eines am Anschlussstück angeordneten Aufnahmeschlitzes, mit welchem ein an der Kupplungseinrichtung angeordneter Kupplungsdorn während des Einschubvorgangs formschlüssig in Eingriff bringbar ist.

Ein Ausführungsbeispiel der Erfindung ist in den Zeichnungen rein schematisch dargestellt und wird nachfolgend näher beschrieben. Es zeigt
- Figur 1: einen Ausschnitt einer Durchschub-Spülmaschine 1 mit einem Korb 4;
- Figur 2: ausschnittsweise das an einem Korb 4 befestigte Anschlussstück 7
- Figur 3: einen Querschnitt durch eine Kupplungseinrichtung 10 mit Anschlussstück 7;
- Figur 4: die Kupplungseinrichtung 10 mit Anschlussstück 7 nach Figur 2 in perspektivischer Darstellung

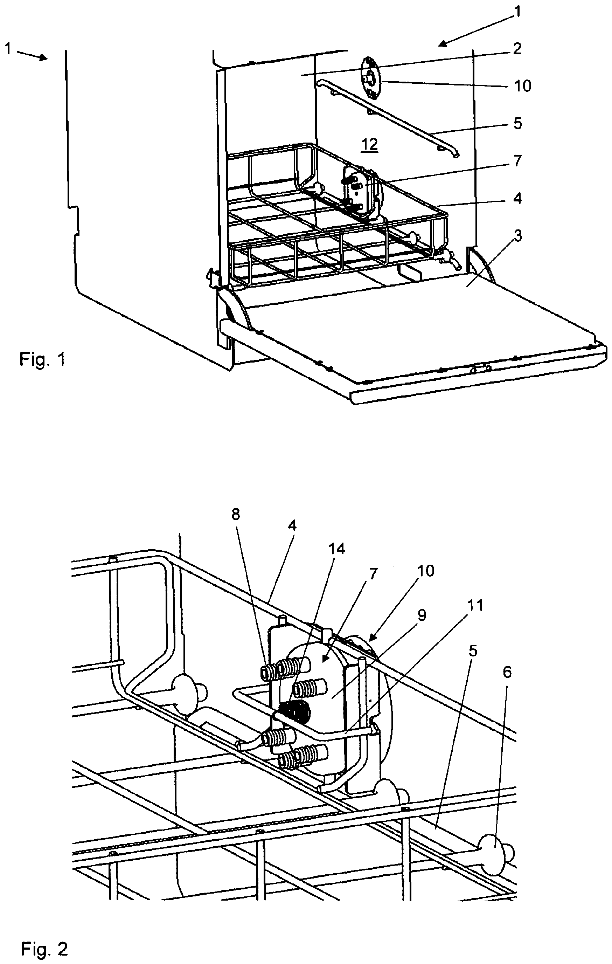

In der Figur 1 ist der Spülraum 2 einer erfindungsgemäß aufgebauten Spülmaschine 1 gezeigt. Das Gerät ist als sogenannte Durchschub-Spülmaschine konzipiert, bei der sowohl die Vorderals auch die Rückseite des Spülraums 2 durch eine Tür verschlossen ist. Die Zeichnung zeigt die schwenkbare Vordertür 3. Das gezeigte Ausführungsbeispiel fungiert als Reinigungs- und Desinfektionsautomat für in den Zeichnungen nicht dargestellte Endoskope, denkbar sind auch andere Einsatzfälle, in denen schlauchförmiges Spülgut gereinigt und/oder desinfiziert wird.

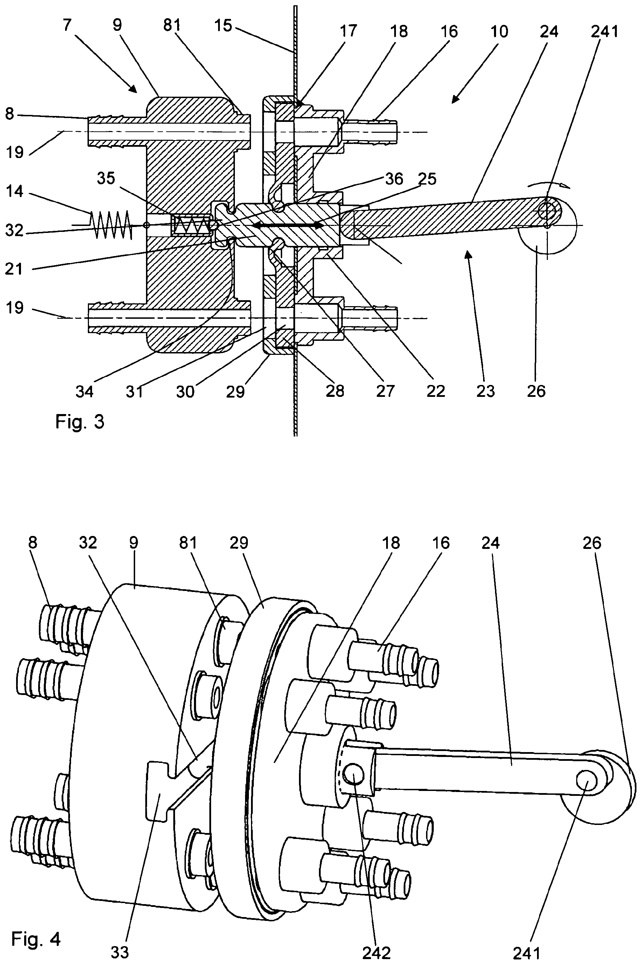

Zur Halterung der Endoskope sind in bekannter Weise Körbe 4 vorgesehen, welche auf seitlichen Schienen 5 durch die Vordertür 3 in den Spülraum 2 eingeschoben und durch die Hintertür aus dem Spülraum 2 entnommen werden. Hierzu sind die Körbe 4 mit Gleitstücken 6 ausgestattet. Die Endoskope besitzen mehrere Kanäle, die auch von innen gereinigt, desinfiziert und getrocknet werden sollen. Deshalb ist an jedem Korb 4 ein Anschlussstück 7 angeordnet, über welches die Endoskopkanäle maschineninterne Einrichtungen zur Speisung mit Reinigungs- und Desinfektionsflotte, Trocknungsluft und Druckluft (zur Dichtigkeitsüberprüfung) angeschlossen werden können. Diese Einrichtungen sind beispielsweise aus der EP 0 483 059 B1 hinreichend bekannt und deshalb hier nicht näher beschrieben. Zu dem vorgenannten Zweck besitzt das in den Figuren 2 und 3 näher gezeigte Anschlussstück 7 sechs Anschlussstutzen 8, die in einem Träger 9 angeordnet sind. Die Anschlussstutzen 8 werden vor dem Einschieben des Korbs vom Benutzer mit den jeweiligen Endoskopkanälen verbunden. Der Träger 9 fungiert einerseits als Halterung für die Stutzen 8, andererseits ermöglicht er die Kopplung mit einer später beschriebenen maschinenseitigen Kupplungsvorrichtung 10. Hierzu ist der Träger 9, wie in Figur 2 näher zu erkennen ist, mitsamt den Stutzen 8 beweglich an einem Haltebügel 11 am Korb 4 befestigt, und zwar so, dass die Bewegungsrichtung quer zur Einschubrichtung des Korbs 4 zur Kupplungsvorrichtung 10 hin verläuft. Durch die Feder 14 wird das Anschlussstück 7 am Haltebügel 11 in der Ausgangslage gehalten. Im gezeigten Ausführungsbeispiel wird eine Zugfeder verwendet, die Verwendung einer Druckfeder oder eines Elastomerteils wäre ebenfalls denkbar. Anstelle der beweglichen Lagerung des Anschlussstücks 7 relativ zum Korb 4 könnte der gesamte Korb 4 seitlich verschiebbar innerhalb des Spülraums angeordnet sein (nicht dargestellt). Hierzu müssten entweder die Gleitstücke 6 oder die Schienen 5 in Richtung Spülraumseitenwand 15 beweglich gelagert sein.

Maschinenseitig ist in der Spülraumseitenwand 15 für jede Korbebene eine Kupplungseinrichtung 10 vorgesehen. Die in den Figuren 3 und 4 näher gezeigte Kupplungseinrichtung 10 umfasst insgesamt sechs Kupplungsstutzen 16, die einzelne Öffnungen 17 in der Spülraumwand 15 durchragen. Die Kupplungsstutzen 16 sind zu einem einstückigen Kupplungsstück 18 zusammengefasst. Bei eingeschobenem Korb 4 fluchten die Anschlussstutzen 8 und die Kupplungsstutzen 16 der jeweiligen Korbebene, in Figur 3 ist dies durch gemeinsame Achslinien 19 symbolisiert. In der Mitte zwischen den Kupplungsstutzen 16 besitzt die Spülraumseitenwand 15 eine weitere Durchtrittsöffnung 20, durch die ein Kupplungsdorn 21 ins Spülrauminnere ragt. Der Dorn 21 ist in einem mittigen Führungsstutzen 22 des Kupplungsstücks 18 gelagert und kann durch die Pleuelstange 24 eines Kurbeltriebs 23 in Richtung des Doppelpfeils 25 bewegt werden. Beide Figuren 3 und 4 zeigen die Kurbelscheibe 26 in einer "neutralen" Stellung, in der sich das kurbelseitige Ende 241 der Pleuelstange 24 in 12-Uhr-Stellung befindet. Zur Abdichtung der Durchtrittsöffnung 20 ist der Kupplungsdorn 21 von einer Membran 27 umgeben, die eine lineare Bewegung in die später beschriebenen Endstellungen zulässt. Die Membran 27 ist zu einer Dichtfläche 28 erweitert, die auf der Innenseite der Spülraumseitenwand 15 vor den Enden der Kupplungsstutzen 16 liegt und durch einen Haltering befestigt wird. Da die Dichtfläche 27 im Bereich der Kupplungsstutzen 16 Öffnungen 30 besitzt, deren Durchmesser geringer ist als der Innendurchmesser der Stutzen 16, stellt sie für jedes kupplungsseitige Ende 81 eines Anschlussstutzens 8 eine axiale Dichtung dar. Auf der Innenseite der Spülraumseitenwand 15 sind die Enden der Kupplungsstutzen 16 und die Membran 27 bzw. Dichtfläche 28 von dem Halterring 29 umgeben, welcher Durchbrüche 31 zur formschlüssigen Aufnahme der kupplungsseitigen Enden 81 der Anschlussstutzen 8 aufweist.

Das spülraumseitige Ende des Kupplungsdorns 21 ist mit einem Pilzkopf 32 versehen, der mit einem Aufnahmeschlitz 33 am Träger 9 des Anschlussstücks 7 zusammenwirkt. Beim Einschieben des Korbs 4 gleitet der Schlitz 33 über die Verjüngung 34 vor den Pilzkopf 32 und stellt eine formschlüssige Verbindung her. Damit eine sichere Positionierung der Stutzen 8 und 16 zueinander gewährleistet ist, ist ein federbelastetes Rastelement 35 vorgesehen, welches in eine Vertiefung 36 im Pilzkopf 32 eingreift. Ist diese Position erreicht, wird nach üblichen Sicherheitsabfragen (Türen geschlossen, Programm gestartet, etc.) ein in den Zeichnungen nicht dargestellter Schrittmotor von der Programmsteuerung (ebenfalls nicht dargestellt) eingeschaltet und dreht die Kurbelscheibe 26 derart, dass das dort befestigte Ende 241 der Pleuelstange 24 von der Neun-Uhr-Position in die Drei-Uhr-Position wandert. Hierdurch wird der Kupplungsdorn 21 in den Figuren 3 und 4 nach rechts gezogen und das formschlüssig mit dem Dorn 21 verbundene Anschlussstück 7 in eine Position bewegt, in der die Enden 81 der Anschlussstutzen 8 an den Öffnungen 30 der Dichtfläche 28 anliegen. Damit ist die luft- und flüssigkeitsdichte Verbindung zwischen den Anschlussstutzen 8 und den Kupplungsstutzen 9 hergestellt, eine Beaufschlagung der Endoskopkanäle mit Flüssigkeit und Luft kann erfolgen.

Das Gerät kann, wie in Figur 1 angedeutet, mit mehreren Kupplungsvorrichtungen 10 zum Reinigen in mehreren Ebenen ausgerüstet sein. Sämtliche Vorrichtungen werden von einer oder mehreren Umwälzpumpen versorgt. Beim Anschluss von Endoskopen wird prinzipiell an jeden angeschlossenen Kanal ein hydraulischer Widerstand angebracht, da jeder Endoskopkanal einen geringeren Querschnitt besitzt als die maschineninternen Zuführungsleitungen (nicht dargestellt). Um bei nicht belegter Spülebene keinen hydraulischen Druckverlust zu verursachen, ist in jeder Kupplungsvorrichtung ein in den Zeichnungen nicht dargestelltes Ventil integriert, welches erst bei der Verbindung zwischen Anschlussstück und Kupplungsvorrichtung automatisch geöffnet wird.

## Patentansprüche

1. Spülmaschine (1), insbesondere Reinigungs- und/oder Desinfektionsautomat für schlauchförmiges Reinigungsgut, mit einem Spülraum (2) zur Aufnahme von mindestens einem Spülgutträger (4), und mit einer Kupplungsvorrichtung (10) zur Einleitung von Spül- und oder Desinfektionsflüssigkeit und/oder Trocknungsluft in ein am Spülgutträger (4) angeordnetes Anschlussstück (7),
**gekennzeichnet durch** eine Zugeinrichtung (21, 23) zur Bewegung des angekuppelten Anschlussstücks (7) in Richtung der Kupplungsvorrichtung (10).

2. Spülmaschine (1) nach Anspruch 1,
**dadurch gekennzeichnet,**
**dass** die Zugeinrichtung (21, 23) innerhalb der Kupplungsvorrichtung (10) angeordnet ist.

3. Spülmaschine (1) nach Anspruch 1 oder 2,
**dadurch gekennzeichnet,**
**dass** die Zugeinrichtung (21, 23) einen elektromotorisch oder elektromagnetisch bewegbaren Kupplungsdorn (21) umfasst, welcher mit dem Spülgutträger (4), insbesondere mit dem Anschlussstück (7) kuppelbar ist.

4. Spülmaschine (1) nach Anspruch 3,
**dadurch gekennzeichnet,**
**dass** die Bewegung des Kupplungsdorns (21) durch einen Kurbeltrieb (23) erfolgt.

5. Spülmaschine (1) nach Anspruch 3 oder 4,
**dadurch gekennzeichnet,**
**dass** der Kupplungsdorn (21) eine Spülraumwand (15) wenigstens teilweise durchragt.

6. Spülmaschine (1) nach Anspruch 5,
**dadurch gekennzeichnet,**
**dass** der Kupplungsdorn (21) von einer Membran (27) umgeben ist, welche die Durchtrittsöffnung (20) in der Spülraumwand abdichtet.

7. Spülmaschine (1) nach Anspruch 6,
**dadurch gekennzeichnet,**
**dass** die Membran (27) zu einer Dichtfläche (28) erweitert ist, die an der Spülrauminnenseite vor den Enden von Kupplungsstutzen (16) der Kupplungsvorrichtung (10) liegt.

8. Spülmaschine (1) nach mindestens einem der vorhergehenden Ansprüche,
**dadurch gekennzeichnet,**
**dass** die Kupplungsvorrichtung (10) in einer Seitenwand (15) des Spülraums (2) angeordnet ist.

9. Spülmaschine (1) nach mindestens einem der vorhergehenden Ansprüche,
**gekennzeichnet durch** eine Vorrichtung zur Verschiebung des gesamten Spülgutträgers (4) in Richtung der Kupplungsvorrichtung (10).

10. Spülgutträger (4) zum Einschub in den Spülraum (2) einer Spülmaschine (1), insbesondere eines Reinigungs- und/oder Desinfektionsautomaten für schlauchförmiges Reinigungsgut, mit einem Anschlussstück (7) zur Verbindung mit einer Kupplungsvorrichtung (10) der Spülmaschine (1) zur Einleitung von Spül- und oder Desinfektionsflüssigkeit und/oder Trocknungsluft, wobei das Anschlussstück (7) relativ zu seinem Befestigungsbereich am Spülgutträger (4) beweglich angeordnet ist,
**dadurch gekennzeichnet,**
**dass** das Anschlussstück (7) quer zur Einschubrichtung des Spülgutträgers (4) zur Kupplungsvorrichtung (10) hin beweglich ist.

11. Spülgutträger (4) nach Anspruch 10,
**gekennzeichnet durch** einen am Anschlussstück (7) angeordneten Aufnahmeschlitz (33), mit welchem ein an der Kupplungseinrichtung (10) angeordneter Kupplungsdorn (21) während des Einschubvorgangs formschlüssig in Eingriff bringbar ist.

12. System, bestehend aus einer Spülmaschine (1) nach mindestens einem der Ansprüche 1 bis 9 und einem Spülgutträger (4) nach mindestens einem der Ansprüche 10 oder 11.

## Claims

1. Dishwasher (1), in particular an automatic cleaning and/or disinfection machine for tubular items to be cleaned, comprising a washing chamber (2) for receiving at least one carrier (4), and a coupling device (10) for introducing rinsing and/or disinfection liquid and/or drying air into a connecting piece (7) arranged on the carrier (4),
**characterised by** a traction means (21, 23) for moving the coupled connecting piece (7) towards the coupling device (10).

2. Dishwasher (1) according to claim 1,
**characterised in that**
the traction means (21, 23) is arranged inside the coupling device (10).

3. Dishwasher (1) according to either claim 1 or claim 2,
**characterised in that**
the traction means (21, 23) comprises an elecromotively or electromagnetically movable coupling mandrel (21) which can be coupled to the carrier (4), in particular to the connecting piece (7).

4. Dishwasher (1) according to claim 3,
**characterised in that**
the coupling mandrel (21) is moved by a crank mechanism (23).

5. Dishwasher (1) according to either claim 3 or claim 4,
**characterised in that**
the coupling mandrel (21) projects through a washing chamber wall (15) at least in part.

6. Dishwasher (1) according to claim 5,
**characterised in that**
the coupling mandrel (21) is surrounded by a membrane (27) which seals the through-opening (20) in the washing chamber wall.

7. Dishwasher (1) according to claim 6,
**characterised in that**
the membrane (27) widens to form a sealing surface (28) which is located on the inside of the washing chamber, in front of the ends of the coupling connecting pieces (16) of the coupling device (10).

8. Dishwasher (1) according to at least one of the preceding claims,
**characterised in that**
the coupling device (10) is arranged in a side wall (15) of the washing chamber (2).

9. Dishwasher (1) according to at least one of the preceding claims,
**characterised by** a device for moving the entire carrier (4) towards the coupling device (10).

10. Carrier (4) for insertion into the washing chamber (2) of a dishwasher (1), in particular an automatic cleaning and/or disinfection machine for tubular items to be cleaned, which carrier comprises a connecting piece (7) for connecting to a coupling device (10) of the dishwasher (1) in order to introduce rinsing and/or disinfection liquid and/or drying air, the connecting piece (7) being arranged on the carrier (4) so as to be movable relative to the attachment region thereof, **characterised in that**
the connecting piece (7) can be moved towards the coupling device (10), transversely to the insertion direction of the carrier (4).

11. Carrier (4) according to claim 10,
**characterised by** a receiving slot (33) which is arranged on the connecting piece (7) and with which a coupling mandrel (21) arranged on the coupling device (10) can be brought into positive engagement during the insertion process.

12. System, consisting of a dishwasher (1) according to at least one of claims 1 to 9, and a carrier (4) according to at least one of claims 10 or 11.

## Revendications

1. Lave-vaisselle (1), en particulier machine automatique de nettoyage et/ou de désinfection pour un article à nettoyer en forme de tuyau, avec un espace de lavage (2) destiné à recevoir au moins un support d'article à nettoyer (4), et avec un dispositif d'accouplement (10) destiné à l'introduction de liquide de lavage et/ou de désinfection et/ou d'air de séchage dans une pièce de raccordement (7) disposée sur le support d'article à nettoyer (4),
**caractérisée par** un dispositif de traction (21, 23) destiné au mouvement de la pièce de raccordement (7) accouplée en direction du dispositif d'accouplement (10).

2. Lave-vaisselle (1) selon la revendication 1,
**caractérisée en ce que**
le dispositif de traction (21, 23) est disposé à l'intérieur du dispositif d'accouplement (10).

3. Lave-vaisselle (1) selon la revendication 1 ou 2,
**caractérisée en ce que**
le dispositif de traction (21, 23) comprend un mandrin d'accouplement (21), pouvant être déplacé par moteur électrique ou par électroaimant, qui peut être couplé au support d'article à nettoyer (4), en particulier à la pièce de raccordement (7).

4. Lave-vaisselle (1) selon la revendication 3,
**caractérisée en ce que**
le déplacement du mandrin d'accouplement (21) s'effectue par un mécanisme à manivelle (23).

5. Lave-vaisselle (1) selon la revendication 3 ou 4,
**caractérisée en ce que**
le mandrin d'accouplement (21) traverse au moins partiellement une paroi d'espace de lavage (15).

6. Lave-vaisselle (1) selon la revendication 5,
**caractérisée en ce que**
le mandrin d'accouplement (21) est entouré d'une membrane (27) qui assure l'étanchéité de l'orifice de passage (20) dans la paroi d'espace de lavage.

7. Lave-vaisselle (1) selon la revendication 6,
**caractérisée en ce que**
la membrane (27) est élargie pour former une surface d'étanchéité (28) qui est située sur le côté intérieur d'espace de lavage devant les extrémités de tubulures d'accouplement (16) du dispositif d'accouplement (10).

8. Lave-vaisselle (1) selon au moins l'une des revendications précédentes,
**caractérisée en ce que**
le dispositif d'accouplement (10) est disposé dans une paroi latérale (15) de l'espace de lavage (2).

9. Lave-vaisselle (1) selon au moins l'une des revendications précédentes,
**caractérisée par** un dispositif destiné à faire coulisser la totalité du support d'article à nettoyer (4) en direction du dispositif d'accouplement (10).

10. Support d'article à nettoyer (4) destiné l'insertion dans l'espace de lavage (2) d'une lave-vaisselle (1), en particulier d'une machine automatique de nettoyage et/ou de désinfection pour un article à nettoyer en forme de tuyau, avec une pièce de raccordement (7) destinée au raccordement à un dispositif d'accouplement (10) de la lave-vaisselle (1) pour l'introduction de liquide de lavage et/ou de désinfection et/ou d'air de séchage, dans lequel la pièce de raccordement (7) est disposée de façon mobile par rapport à sa zone de fixation sur le support d'article à nettoyer (4),
**caractérisé en ce que**
la pièce de raccordement (7) est mobile transversalement à la direction d'insertion du support d'article à nettoyer (4) en direction du dispositif d'accouplement (10).

11. Support d'article à nettoyer (4) selon la revendication 10,
**caractérisé par** une fente de réception (33) qui est disposée sur la pièce de raccordement (7) et avec laquelle un mandrin d'accouplement (21) disposé sur le dispositif d'accouplement (10) peut être mis en prise par liaison de forme pendant le processus d'insertion.

12. Système, composé d'une lave-vaisselle (1) selon au moins l'une des revendications 1 à 9 et d'un support d'article à nettoyer (4) selon au moins l'une des revendications 10 ou 11.
